(19) Europäisches Patentamt
European Patent Office
Office européen des brevets

(11) Publication number: **0 148 004 A2**

(12) **EUROPEAN PATENT APPLICATION**

(21) Application number: 84308942.6

(22) Date of filing: 20.12.84

(51) Int. Cl.[4]: C 07 D 501/20
C 07 D 277/20, A 61 K 31/545

(30) Priority: 30.12.83 GB 8334599
30.12.83 GB 8334600

(43) Date of publication of application:
10.07.85 Bulletin 85/28

(84) Designated Contracting States:
AT BE CH DE FR GB IT LI LU NL SE

(71) Applicant: GLAXO GROUP LIMITED
Clarges House 6-12 Clarges Street
London W1Y 8DH(GB)

(72) Inventor: Newall, Christopher Earle
33 Elm Grove Ealing
London W5(GB)

(72) Inventor: Foxton, Michael Walter
Mill Lane The Finches
Chalfont St. Giles Buckinghamshire(GB)

(72) Inventor: Hartley, Charles David
48 Sycamore Close
Northolt Middlesex(GB)

(72) Inventor: Looker, Brian Edgar
28 Middleton Avenue
Greenford Middlesex(GB)

(74) Representative: Holmes, Michael John et al,
Frank B. Dehn & Co. European Patent Attorneys Imperial
House 15-19 Kingsway
London, WC2B 6UZ,(GB)

(54) **Cephalosporin antibiotics.**

(57) Cephalosporin compounds of general formula

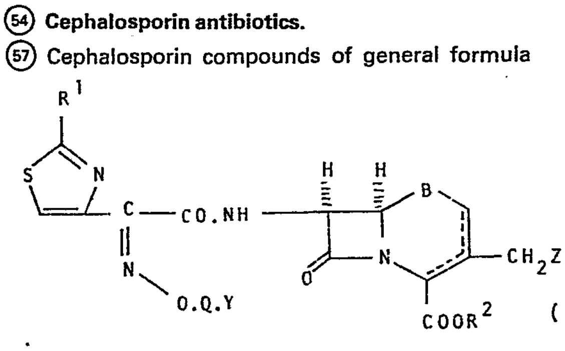

(I)

(wherein

Q represents a cyclopropylmethyl goup,

Y represents an optionally blocked carboxyl group or an optionally N-mono- or N,N-di-alkyl substituted carbamoyl group,

Z represents hydrogen, optionally N-substituted carbamoyloxy, heterocyclicthio or optionally substituted pyridinium or bicyclicpyridinium,

R represents an optionally protected amino group,

$R^2$ represents a hydrogen atom or a carboxyl blocking group,

B represents –S– or >S→O, and the dotted line indicates $\Delta^2$ or $\Delta^3$ unsaturation) and salts thereof.

The $\Delta^3$ sulphide compounds exhibit a particularly advantageous profile of antibiotic activity against both Gram-positive and Gram-negative and organisms, including β-lactamase-producing strains.

Methods of preparing the compounds of the invention are described, as is the use of the compounds as active antibiotics and as precursors therefor.

EP 0 148 004 A2

0148004

FE 146-990

# Cephalosporin Antibiotics

This invention relates to improvements in or relating to cephalosporins. More particularly it relates to new cephalosporin compounds and derivatives thereof having valuable antibiotic activity.

The cephalosporin compounds in this specification are named with reference to "cepham" after J.Amer.Chem. Soc., 1962, 84, 3400, the term "cephem" referring to the basic cepham structure with one double bond.

Cephalosporin antibiotics are widely used in the treatment of diseases caused by pathogenic bacteria in human beings and animals, and are especially useful in the treatment of diseases caused by bacteria which are resistant to other antibiotics such as penicillin compounds, and in the treatment of penicillin-sensitive patients. In many instances it is desirable to employ a cephalosporin antibiotic which exhibits activity against both Gram-positive and Gram-negative microorganisms, and a significant amount of research has been directed to the development of various types of broad spectrum cephalosporin antibiotics.

Thus, for example in our British Patent Specification No. 1399086, we describe a novel class of cephalosporin antibiotics containing a 7β-(α-etherified oxyimino)acylamido group, the oxyimino group having the *syn* configuration. This class of antibiotic compounds is characterised by high antibacterial activity against a range of Gram-positive and Gram-negative organisms coupled with particularly high stability to β-lactamases produced by various Gram-negative organisms.

The discovery of this class of compounds has stimulated further research in the same area in attempts to find compounds which have improved properties, for example against particular classes

of organisms especially Gram-negative organisms. This interest is reflected in the very large numbers of patent applications which have been filed relating to cephalosporin antibiotics having particular oxyimino etherifying groups in combination with particular substituents both on the 7β-acylamido side chain and at the 3-position of the cephalosporin nucleus.

British Patent Specification No. 1604971 generically defines a wide variety of cephalosporin compounds in which the 7β-side chain may be selected from, <u>inter</u> <u>alia</u>, a 2-(2-aminothiazol-4-yl)-2-(etherified oxyimino)acetamido group in which the etherifying group, amongst very many possible meanings, may be a substituted methyl group, although the preferred etherifying group is an unsubstituted methyl group. The methyl group substituents, where present, may include <u>inter</u> <u>alia</u> a cycloalkyl, carboxyl, alkoxycarbonyl, carbamoyl or cyano group. The 3-position group may also be selected from a large number of alternatives.

European Patent Application No. 88320 contains a generic definition of a wide variety of cephalosporin antibiotics in which the 7β-side chain may be selected from, <u>inter</u> <u>alia</u>, a 2-(2-aminothiazol-4-yl)-2-(etherified oxyimino)acetamido group. The 3-position group is an optionally substituted pyridiniummethyl group. The definition of the oxyimino etherifying group includes, among very many possibilities, cycloalkylmethyl which may be substituted at the 1-position of the cycloalkyl ring by a substituent selected from carboxyl, alkoxycarbonyl, cyano and optionally substituted carbamoyl. However, there is no specific exemplification of compounds having such groups, and in particular no reference to a cyclopropylmethyl etherifying group carrying such substituents.

We have now discovered that by the selection of a (Z)-2-(etherified oxyimino)acetamido group at the 7β-position (the said group being substituted at the 2-position thereof by a 2-aminothiazol-4-yl group) in combination with certain particular groups as herein defined at the 3-position of the cephalosporin nucleus, and by the selection of a cyclopropylmethoxyimino group substituted by a carboxy, alkoxycarbonyl, carbamoyl, substituted carbamoyl or cyano group as the etherified oxyimino grouping, cephalosporin compounds having a particularly advantageous profile of activity (described in more detail below) again a wide range of commonly encountered pathogenic organisms may be obtained.

According to one aspect of the invention we provide cephalosporin compounds of general formula

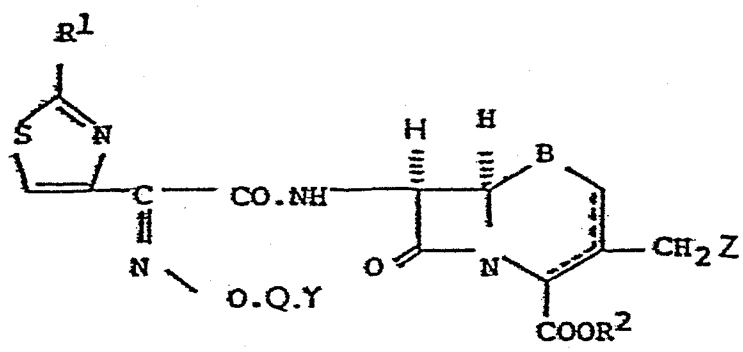

(I)

[wherein

Q represents a cyclopropylmethyl group;

Y represents a carboxyl or blocked carboxyl group, a carbamoyl, N-($C_{1-4}$ alkyl)carbamoyl or N,N-di-($C_{1-4}$ alkyl)carbamoyl group or a cyano group;

Z represents a hydrogen atom; a carbamoyloxy group optionally substituted on the nitrogen atom by a $C_{1-4}$ alkyl or $C_{1-4}$ haloalkyl group; an N-protected carbamoyloxy group; a group of formula $-SZ^a$ (wherein $Z^a$ is a carbon-linked 5- or 6-membered unsaturated

heterocyclic ring containing at least one nitrogen atom, which ring may also contain one or more sulphur atoms and/or may be substituted by one or more substituents independently selected from $C_{1-4}$ alkyl, oxo, hydroxy or carbamoyloxymethyl groups); a pyridinium group which may optionally be substituted by one or more substituents independently selected from halogen atoms and carbamoyl, hydroxymethyl and $C_{1-4}$ alkoxy groups; or a bicyclic pyridinium group of formula

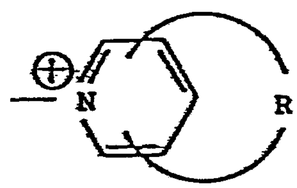

(wherein R represents an optionally substituted $C_{2-5}$ hydrocarbon chain which is saturated or contains one or two double bonds and which may optionally be interrupted by a heteroatom);

$R^1$ represents an amino or protected amino group;

$R^2$ represents a hydrogen atom or a carboxyl blocking group;

B is -S- or $>S\rightarrow O$ (α- or β-); and the dotted line bridging the 2-, 3- and 4-positions indicates that the compound is a ceph-2-em or ceph-3-em compound] and salts thereof.

It will be understood that the term "salt" used herein, both in connection with compounds of general formula I and with intermediates used in their preparation, includes within its scope external and internal salts. External salts may, for example, be base salts or may be acid addition salts formed with e.g. a mineral or organic acid.

Internal salts may for example take the form of zwitterions or betaines. For pharmaceutical use, salts may be non-toxic salts but other salts may find use, for example in the preparation of compounds to be used as pharmaceuticals.

It will be appreciated that the group Z may carry a positive charge, e.g. when Z is a pyridinium or bicyclic pyridinium group or a group of formula $-SZ^a$, and when this is the case the positive charge must be balanced by a negative charge. Thus the compounds may be betaines, so that the negative charge is provided by a $-COO^{\ominus}$ group. Alternatively, the negative charge may be provided by an anion $A^{\ominus}$. In compounds to be used in medicine such an anion will be non-toxic and may be derived from any of the acids described below which will form non-toxic salt derivatives.

The compounds according to the invention are _syn_ isomers. The _syn_ isomeric form is defined by the configuration of the -O.Q.Y group with respect to the carboxamido group. In this Specification, the _syn_ configuration is denoted structurally as

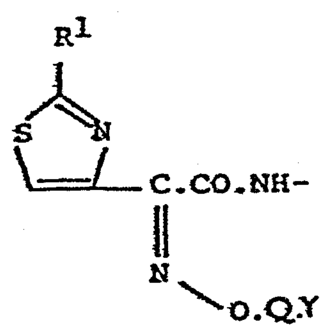

It will be understood that since the compounds according to the invention are geometric isomers, some admixture with the corresponding _anti_ isomer may occur.

The invention also includes within its scope the solvates (especially the hydrates) of the compounds of formula (I). It also includes within its scope the solvates of salts of compounds of formula (I).

It will be appreciated that where the compounds are to be used in medicine, the solvates should be pharmacologically acceptable.

It will be appreciated that the group Q in the compounds of the invention may be substituted by the group Y at any part of the cyclopropylmethyl skeleton. For the avoidance of doubt, the following convention is used herein for identifying the substituent positions on the cyclopropylmethyl group:

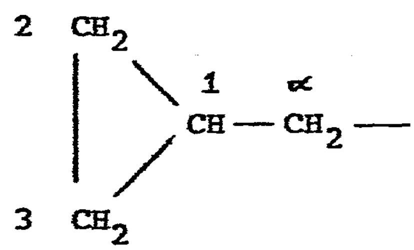

When the carbon atom at the α-position carries a substituent, the carbon atom may be in the R or the S configuration. The invention covers the individual enantiomeric forms as well as mixtures, including racemic mixtures.

When the group Y in the compounds of the invention represents a blocked carboxyl group this may for example be an esterified carboxyl group, as discussed in more detail below.

When the group Z represents a group of formula $-SZ^a$, the heterocyclic ring represented by $Z^a$ may for example contain 1 to 4 nitrogen atoms and, if desired, a sulphur atom; particular examples of these heterocyclic groups include imidazolyl, pyrazolyl, pyridyl, pyrimidyl, pyrazinyl, pyridazinyl, triazolyl, tetrazolyl, thiazolyl, thiadiazolyl, triazinyl and thiazolidinyl. The heterocyclic ring may, if desired, be substituted by one or more (for example one to three) $C_{1-4}$ alkyl (e.g. methyl), oxo, hydroxy or carbamoylmethyl groups. Alkyl or carbamoylmethyl substituents may be attached for example, to the nitrogen heteroatom(s). In

the heterocyclic ring represented by $Z^a$ one nitrogen atom may be quaternised.

When Z represents a bicyclic pyridinium group in which the $C_{2-5}$ hydrocarbon chain is interrupted by a heteroatom, the said heteroatom may, for example, by an oxygen, nitrogen or sulphur atom. When Z represents a bicyclic pyridinium group, this may for example be a 2,3-cyclopentenopyridinium group.

One group of compounds of formula (I) of interest is the group of compounds in which Z represents an optionally substituted pyridinium group or a bicyclic pyridinium group as defined above with the exception of compounds in which Z represents an optionally substituted quinolinium or isoquinolinium group, and the cyclopropylmethyl group Q carries substituent Y at the 1-position.

When the group Z in the compounds of the invention represents an N-protected carbamoyloxymethyl group, this group Z may be a group of formula $-O.CO.NHR^3$ where $R^3$ is a conventional N-protecting group such as, for example, a labile group such as an acyl group, especially a lower alkanoyl group such as acetyl, a halo-substituted lower alkanoyl group such as mono-, di- or tri-chloroacetyl, a chloro- or bromo-sulphonyl group, or a halogenated alkoxycarbonyl group such as 2,2,2-trichloroethoxycarbonyl.

When the group $R^1$ in the compounds of the invention represents a protected amino group, this may for example be an amino group protected using a conventional protective group, as discussed in more detail below.

When the group $R^2$ in the compounds of the invention represents a carboxyl blocking group, this, or the carboxyl blocking group in Y when this group represents a blocked carboxyl group, may for example be the residue of an ester-forming aliphatic or araliphatic alcohol or an ester-forming

phenol, silanol or stannanol (the said alcohol, phenol, silanol or stannanol preferably containing 1 to 20 carbon atoms) or a symmetrical or mixed anhydride blocking group derived from an appropriate acid. Where the compound is to be used in medicine any ester function $R^2$ should be a non-toxic metabolically labile ester function, as discussed in more detail below.

Certain ceph-3-em compounds of formula (I) and the non-toxic salts thereof, exhibit interesting biological properties (as described in detail below) and are therefore preferred compounds of the invention. These compounds may be represented by the general formula

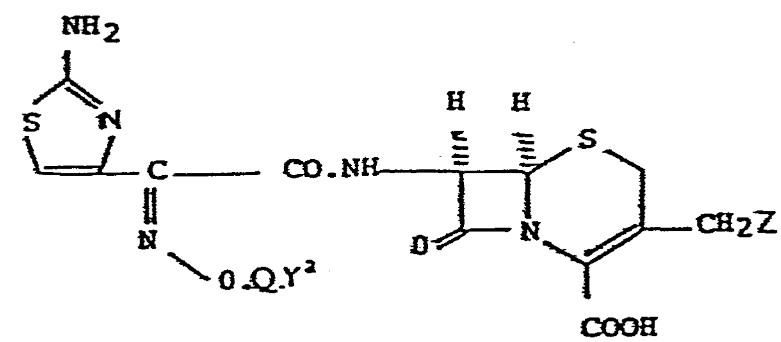

(Ia)

(wherein Q and Z are as defined above, and $Y^a$ is a carboxyl, $C_{2-5}$ alkoxycarbonyl, carbamoyl, N-($C_{1-4}$ alkyl)carbamoyl, N,N-di($C_{1-4}$alkyl)carbamoyl or cyano group) and non-toxic salts (including zwitterionic and betaine forms) and non-toxic metabolically labile esters thereof. Other compounds according to the invention, e.g. compounds in which $R^1$ is a protected amino group, may however be intermediates useful in the preparation of the active compounds.

The preferred compounds of general formula Ia as defined above include those in which Z represents a pyridinium group.

Intermediates such as e.g. ceph-2-em, sulphoxide and protected intermediates are useful in the preparation of the active compounds of the invention.

The compounds according to the present invention may exist in tautomeric forms (for example in respect of the 2-aminothiazolyl group) and it will be understood that such tautomeric forms are included within the scope of the invention.

Examples of non-toxic metabolically labile ester derivatives include acyloxyalkyl esters, e.g. lower alkanoyloxy-methyl or -ethyl esters such as acetoxy-methyl or -ethyl or pivaloyloxymethyl esters, and alkoxycarbonyloxyalkyl esters, e.g. lower alkoxycarbonyloxyethyl esters such as the ethoxycarbonyloxyethyl ester. In addition to the above ester derivatives, the present invention includes within its scope the active compounds of the invention in the form of other physiologically acceptable equivalents, i.e. physiologically acceptable compounds which, like the metabolically labile esters, are converted _in vivo_ into the parent antibiotic compounds of the invention.

Non-toxic salt derivatives which may be formed by reaction of any carboxyl group present in the compounds of formula (I), or by reaction of any acidic hydroxyl group which may be present on the group Z, include inorganic base salts such as alkali metal salts (e.g. sodium and potassium salts) and alkaline earth metal salts (e.g. calcium salts); amino acid salts (e.g. lysine and arginine salts) and other organic base salts (e.g. procaine, phenylethylbenzylamine, dibenzylethylenediamine, ethanolamine, diethanolamine and N-methylglucosamine salts). Other non-toxic salt derivatives include acid addition salts, e.g. formed with hydrochloric, hydrobromic, sulphuric, nitric, phosphoric, formic and trifluoroacetic acids. Where appropriate, the compounds may also, as indicated above, be in zwitterionic i.e. internal salt form. The salts may also be in the form of resinates formed with, for example, a polystyrene resin or cross-linked polystyrene divinylbenzene

copolymer resin containing amino or quaternary amino groups or sulphonic acid groups, or with a resin containing carboxyl groups, e.g. a polyacrylic acid resin. Soluble base salts (e.g. alkali metal salts such as the sodium salt) of the compounds of formula (Ia) may be used in therapeutic applications because of the rapid distribution of such salts in the body upon administration. Where, however, insoluble salts of compounds (Ia) are desired in a particular application, e.g. for use in depot preparations, such salts may be formed in conventional manner, for example with appropriate organic amines.

Active compounds according to the invention exhibit broad spectrum antibiotic activity both in vitro and in vivo. They have good activity against both Gram-positive and Gram-negative organisms, including many β-lactamase producing strains. The compounds also possess high stability to β-lactamases produced by a range of Gram-negative and Gram-positive organisms.

Active compounds according to the invention have been found to exhibit good activity against strains of Staphylococcus aureus and Staphylococcus epidermidis species including penicillinase producing strains of these Gram-positive bacteria. This is coupled with good activity against various members of the Enterobacteriaceae (e.g. strains of Escherichia coli, Klebsiella pneumoniae, Enterobacter cloacae, Serratia marcescens, Proteus mirabilis and indole-positive Proteus organisms such as Proteus vulgaris, Proteus morganii and Providence species), and strains of Haemophilus influenzae and Acinetobacter calcoaceticus as well as activity against some strains of Pseudomonas species. This combination of high activity against Gram-positive organisms with high activity against Gram-negative organisms possessed by compounds of the invention is unusual and particularly advantageous.

Active compounds of the invention may be used for treating a variety of diseases caused by pathogenic bacteria in human beings and animals, such as resporatory tract infections and urinary tract infections.

According to another embodiment of the invention we provide a process for the preparation of cephalosporin compounds of general formula (I) and salts thereof, which comprises

(A) acylating a compound of formula (II)

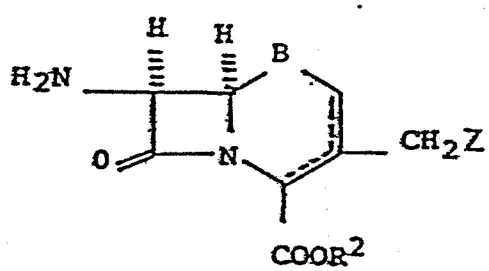

(II)

(wherein Z, B, the dotted line and $R^2$ are as defined above) or a salt (including zwitterionic and betaine forms) thereof, e.g. an acid addition salt (formed with, for example, a mineral acid such as hydrochloric, hydrobromic, sulphuric, nitric or phosphoric acid or an organic such as methanesulphonic or toluene-4-sulphonic acid) or a 7-N-silyl derivative thereof, with an acid of formula (III)

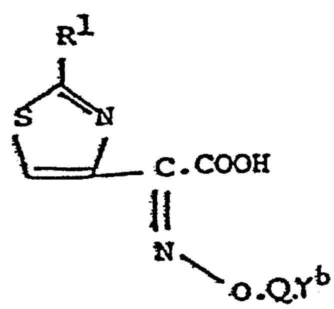

(III)

(wherein $R^1$ and Q are as defined above, and $Y^b$ is as defined above for Y other than a carboxyl group) or a salt thereof, or with an acylating agent corresponding thereto;

(B) where Z in the compound of the invention represents an optionally substituted carbamoyloxy group, reacting a compound of formula (IV)

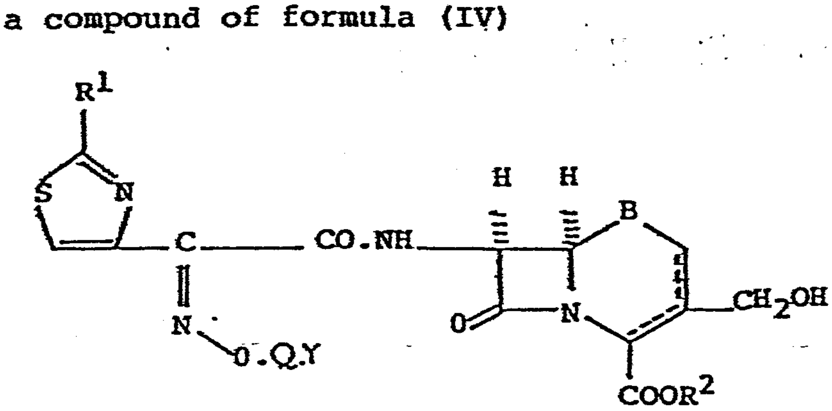

(IV)

(where Q, Y, $R^1$, $R^2$, B and the dotted line are as defined above) or a salt thereof, with an acylating agent serving to form the group $-CH_2Z^b$ at the 3-position (wherein $Z^b$ is a carbamoyloxy group optionally substituted on the nitrogen atom by a $C_{1-4}$ alkyl or $C_{1-4}$ haloalkyl group or an N-protected carbamoyloxy group such as a group of formula $-O.CO.NHR^3$ where $R^3$ is an N-protecting group, e.g. a labile group as described above);

(C) where Z in the compound of the invention represents an optionally substituted pyridinium or bicyclic pyridinium group or a group of formula $-SZ^a$ (where $Z^a$ is as defined above), reacting a compound of formula (V)

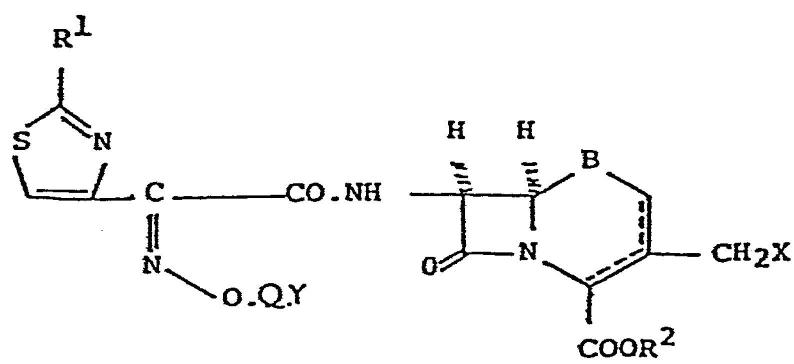

(V)

(wherein Q, Y, $R^1$, $R^2$, B and the dotted line are as defined above, and X represents a replaceable residue of a nucleophile e.g. an acetoxy or dichloroacetoxy group or a halogen atom such as chlorine, bromine or iodine) or a salt thereof, with a nucleophile serving to form the desired group of formula $-CH_2Z$ at the 3-position; or

(D) where Z in the compound of the invention represents a group of formula $-SZ^a$ wherein $Z^a$ contains a $C_{1-4}$ alkyl-substituted or carbamoylmethyl-substituted nitrogen atom in the heterocyclic ring, reacting a compound of formula (Ib)

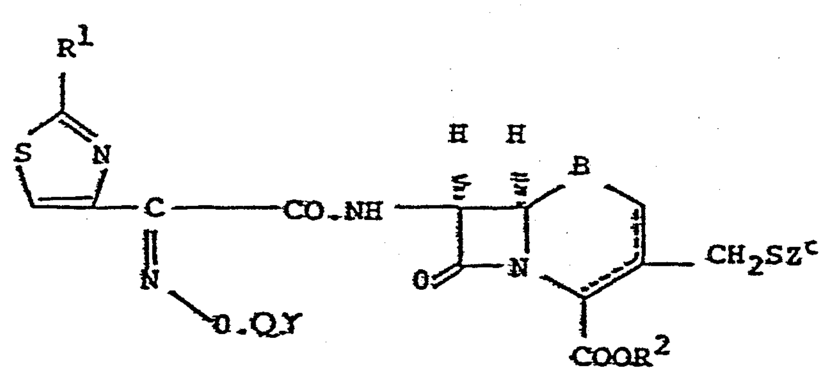

(Ib)

(wherein Q, Y, $R^1$, B and the dotted line are as defined in claim 1; $R^2$ in this instance represents a carboxyl blocking group; and $Z^c$ represents a carbon-linked 5- or 6-membered heterocyclic ring containing a nitrogen atom) with a $C_{1-4}$ alkylating agent or a carbamoylmethylating agent serving to introduce a $C_{1-4}$ alkyl group or a carbamoylmethyl group as a substituent on the nitrogen atom in the heterocyclic ring of $Z^c$.

After any of the above processes (A) to (D) any of the following reactions, in any appropriate sequence, may be carried out to give compounds of formula (Ia) or non-toxic salts or non-toxic metabolically labile esters thereof:-

i) conversion of a $\Delta^2$-isomer into a $\Delta^3$-isomer,

ii) reduction of a compound wherein B is $>S\rightarrow O$ to form a compound wherein B is -S-,

iii) conversion of a carboxyl group into a non-toxic metabolically labile ester function,

iv) formation of a non-toxic salt, and

v) conversion of one group Y into another group Y, e.g. amination of a compound in which Y is a carboxyl or esterified carboxyl group to form a compound in which Y is a carbamoyl or substituted carbamoyl group, dehydration of a compound in which Y is a carbamoyl group to form a compound in which Y is a cyano group, or esterification of a carboxyl group Y, and

vi) removal of any carboxyl blocking and/or N-protecting groups.

In the above-described process (A), the starting material of formula (II) is preferably a compound wherein B is -S- and the dotted line represents a ceph-3-em compound.

When the group Z in formula (II) is charged e.g. a pyridinium group or a group of formula $-SZ^a$ wherein $Z^a$ represents an N-alkyl-pyridinium group, and no free carboxyl group is present in the molecule, the compound will include an associated anion $E^{\ominus}$ such as a halide, e.g. chloride or bromide, or trifluoroacetate anion.

Acylating agents which may be employed in the preparation of compounds of formula (I) include acid halides, particularly acid chlorides or bromides. Such acylating agents may be prepared by reacting an acid (III) or a salt thereof with a halogenating agent e.g. phosphorus pentachloride, thionyl chloride or oxalyl chloride.

Acylations employing acid halides may be effected in aqueous and non-aqueous reaction media, conveniently at temperatures of from -50 to +50°C, preferably -40 to +30°C, if desired in the presence of an acid binding agent. Suitable reaction media include aqueous ketones such as aqueous acetone, aqueous alcohols such as aqueous ethanol, esters such as ethyl acetate, halogenated hydrocarbons such as methylene chloride, amides such as dimethylacetamide, nitriles such as acetonitrile, or mixtures of two or more such solvents. Suitable acid binding agents include tertiary amines (e.g. triethylamine or dimethylaniline), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acids of formula (III) may themselves be used as acylating agents in the preparation of compounds of formula (I). Acylations employing acids (III) are desirably conducted in the presence of a condensing agent, for example a carbodiimide such as N,N'-dicyclohexylcarbodiimide or N-ethyl-N'-γ-dimethylaminopropylcarbodiimide; a carbonyl compound such as carbonyldiimidazole; or an isoxazolium salt such as N-ethyl-5-phenylisoxazolium perchlorate.

Acylation may also be effected with other amide-forming derivatives of acids of formula (III) such as, for example, an activated ester, a symmetrical anhydride or a mixed anhydride (e.g. formed with pivalic acid or with a haloformate, such as a lower alkylhaloformate). Mixed anhydrides may also be formed with phosphorus acids (for example phosphoric or phosphorous acids), sulphuric acid or aliphatic or aromatic sulphonic acids (for example toluene-4-sulphonic acid). An activated ester may conveniently be formed <u>in situ</u> using, for example, 1-hydroxybenzotriazole in the presence of a condensing agent

as set out above. Alternatively, the activated ester may be preformed.

Acylation reactions involving the free acids or their above-mentioned amide-forming derivatives are desirably effected in an anhydrous reaction medium, e.g. methylene chloride, tetrahydrofuran, dimethylformamide or acetonitrile.

An alternative method of activation is, for example, by reacting an acid of formula (III) with a solution or suspension preformed by adding a carbonyl halide, in particular oxalyl chloride or phosgene, or a phosphoryl halide such as phosphorous oxychloride to a solvent such as a halogenated hydrocarbon, for example methylene chloride, containing a lower acyl tertiary amide such as N,N-dimethylformamide. The activated form of the the acid of formula (III) may then be reacted with a 7-amino compound of formula (II) in a suitable solvent or mixture of solvents for example a halogenated hydrocarbon e.g. dichloromethane. The acylation reaction may conveniently be effected at temperatures of from -50° to +50°C, preferably -40° to +30°C, if desired in the presence of an acid binding agent, for example as described above (e.g. dimethylaniline).

If desired, the above acylation reactions may be carried out in the presence of a catalyst such as 4-dimethylaminopyridine.

The acids of formula (III) and acylating agents corresponding thereto may, if desired, be prepared and employed in the form of their acid addition salts. Thus, for example, acid chlorides may conveniently be employed as their hydrochloride salts, and acid bromides as their hydrobromide salts.

Acetylation of 3-hydroxymethyl compounds of formula (IV) according to process (B) may be effected by conventional methods, for example in an analogous manner to that described in British

Patent Specification No. 1141293, i.e. by blocking the 4-carboxy group, acetylating the 3-hydroxymethyl group of the protected compound and subsequently removing the blocking group.

Carbamoylation of 3-hydroxymethyl compounds of formula (IV) may be effected by conventional methods using suitable acylating (i.e. carbamoylating) agents. Suitable carbamoylating agents include isocyanates of formula $R^4$.NCO (wherein $R^4$ is a labile substituent group or the group $R^3$ as defined above), to give a compound containing a 3-position substituent having the formula $-CH_2O.CONHR^4$ (wherein $R^4$ has the above defined meaning). The carbamoylation reaction may desirably be effected in the presence of a solvent or solvent mixture selected from hydrocarbons (e.g. aromatic hydrocarbons such as benzene and toluene), halogenated hydrocarbons (e.g. dichloromethane), amides (e.g. formamide or dimethylformamide), esters (e.g. ethyl acetate), ethers (e.g. cyclic ethers such as tetrahydrofuran and dioxan), ketones (e.g. acetone), sulphoxides (e.g. dimethylsulphoxide) and mixtures of these solvents. The reaction may conveniently be carried out at a temperature of between -80°C and the boiling temperature of the reaction mixture, for example up to 100°C, preferably between -20° and +30°C. The labile group $R^4$ may subsequently be cleaved, e.g. by hydrolysis, to form a 3-carbamoyloxymethyl group. Examples of labile groups $R^4$ which are readily cleavable upon subsequent treatment include those labile groups hereinbefore given as examples of the group $R^3$. Such labile groups may generally be cleaved by acid or base catalysed hydrolysis (e.g. by base catalysed hydrolysis using sodium bicarbonate). Halogenated groups such as chlorosulphonyl, dichlorophosphoryl, trichloroacetyl and 2,2,2-trichloroethoxycarbonyl may also be cleaved reductively, while groups such as chloroacetyl may also be cleaved by treatment with thioamides such as thiourea.

The carbamoylating agent is desirably used in excess (for example at least 1.1 moles relative to the compound of formula (IV)). The carbamoylation may be assisted by the presence of base, e.g. a tertiary organic base such as a tri-(lower alkyl)amine (e.g. triethylamine) or by employing the compound (IV) in the form of an alkali metal (e.g. sodium) salt, although such assistance may not be necessary in the case of more active isocyanates, e.g. compounds when $R^4$ is a strongly electron-withdrawing group such as chlorosulphonyl or trichloroacetyl. Carbamoylations involving reaction of a free acid of formula (IV) with excess isocyanate wherein $R^4$ is a group such as chlorosulphonyl or trichloroacetyl are thus of particular practical advantage by virtue of the simplicity of the reaction conditions, since there is no need for temporary blocking and subsequent deblocking of the 4-position carboxyl group of the cephalosporin and since the electron-withdrawing $R^4$ group in the resulting N-protected 3-carbamoyloxymethyl cephalosporin product is readily removed by, for example, hydrolysis with aqueous sodium bicarbonate.

It should be noted that it may be convenient to retain or even introduce an N-substituting group $R^4$ during transformations of intermediate 3-carbamoyloxymethyl compounds in order to minimise unwanted side reactions involving the carbamoyloxymethyl group.

Another useful carbamoylating agent is cyanic acid, which is conveniently generated in situ, for example, from an alkali metal cyanate such as sodium cyanate, the reaction being facilitated by the presence of an acid, e.g. a strong organic acid such as trifluoroacetic acid. Cyanic acid effectively corresponds to the isocyanate compounds mentioned above wherein $R^4$ is hydrogen and therefore converts compounds of formula (IV) directly to their 3-carbamoyloxymethyl analogues.

Alternatively, carbamoylation may be effected by reaction of the compound of formula (IV) with phosgene or carbonyldiimidazole followed by ammonia or the appropriate substituted amine, optionally in an aqueous or non-aqueous reaction medium.

3-Hydroxymethyl starting materials of formula (IV) are novel compounds and form a further feature of the invention. They may be prepared by methods analogous to those described in British Patent No. 1474519 and U.S. Patent No. 3976546. Alternatively they may be prepared by N-acylating the corresponding 7-amino-3-hydroxymethyl compounds, e.g. analogously to process (A) above.

In process (C) above, the nucleophile may be used to displace a wide variety of substituents X from the cephalosporin of formula (V). To some extent the facility of the displacement is related to the pKa of the acid HX from which the substituent is derived. Thus, atoms or groups X derived from strong acids tend, in general, to be more easily displaced than atoms or groups derived from weaker acids. The facility of the displacement is also related, to some extent, to the precise character of the nucleophile. A sulphur nucleophile for introducing the group -SZ may be employed for example in the form of an appropriate thiol or thione.

The displacement of X by the nucleophile may conveniently be effected by maintaining the reactants in solution or suspension. The reaction is advantageously effected using 1-10 molar equivalents of the nucleophile.

Nucleophilic displacement reactions may conveniently be carried out on those compounds of formula (V) wherein the substituent X is a halogen atom or an acyloxy group, for example as discussed below.

<u>Acyloxy groups</u>

Compounds of formula (V) wherein X is an acetoxy group are convenient starting materials

for use in the nucleophilic displacement reaction with the nucleophile. Alternative starting materials in this class include compounds of formula (V) in which X is the residue of a substituted acetic acid e.g. chloroacetic acid, dichloroacetic acid and trifluoroacetic acid.

Displacement reactions on compounds (V) possessing X substituents in this class, particularly in the case where X is an acetoxy group, may be facilitated by the presence in the reaction medium of iodide or thiocyanate ions. For example, reactions of this type employing pyridine (including bicyclic pyridine) nucleophiles are described in more detail in British Patent Specifications Nos. 1132621 and 1171603 and British Patent Application 2098216A.

The substituent X may also be derived from formic acid, a haloformic acid such as chloroformic acid, or a carbamic acid.

When using a compound of formula (V) in which X represents an acetoxy or substituted acetoxy group, it is generally desirable that the group $R^2$ in formula (V) should be a hydrogen atom and that B should represent -S-. In this case, the reaction is advantageously effected in an aqueous medium. When using a pyridine nucleophile, the aqueous medium is preferably at a pH of 5 to 8, particularly 5.5 to 7.

Under aqueous conditions, the pH value of the reaction solution when employing a sulphur nucleophile is advantageously maintained in the range 6-8, if necessary by the addition of a base. The base is conveniently an alkali metal or alkaline earth metal hydroxide or bicarbonate such as sodium hydroxide or sodium bicarbonate.

A process employing a pyridine nucleophile and a compound of formula (V) in which X is the residue of a substituted acetic acid may be carried out as described in British Patent Specification No. 1241657.

When using compounds of formula (V) in which X is an acetoxy group, the reaction is conveniently effected at a temperature of 10° to 110°C, preferably 20° to 80°C.

Halogens

Compounds of formula (V) in which X is a chlorine, bromine or iodine atom are novel compounds which form a further feature of the invention and can be conveniently used as starting materials in the nucleophilic displacement reaction with the nucleophile. When using compounds of formula (V) in this class, $R^2$ may conveniently represent a carboxyl blocking group. When using a sulphur nucleophile, B in the compound of formula (V) conveniently represents -S-, whereas when using a pyridine nucleophile, B is preferably $>S \rightarrow O$. The reaction is conveniently effected in a non-aqueous medium which preferably comprises one or more organic solvents, advantageously of a polar nature such as ethers, e.g. dioxan or tetrahydrofuran, esters, e.g. ethyl acetate, amides e.g. formamide and N,N-dimethylformamide, and ketones e.g. acetone. In some cases the nucleophile itself may be the solvent. Other suitable organic solvents are described in more detail in British Patent Specification No. 1326531. The reaction medium should be neither extremely acidic nor extremely basic.

In the case of reactions carried out on compounds of formula (V) in which $R^2$ is a carboxyl blocking group and the resulting Y group contains a quaternary nitrogen atom, the product will be formed as the corresponding halide salt which may, if desired, be subjected to one or more ion exchange reactions to obtain a salt having the desired anion.

When using compounds of formula (V) in which X is a halogen atom as described above, the reaction is conveniently effected at a temperature of -20° to +60°, preferably 0° to +30°C.

When the incoming nucleophile does not yield

a compound containing a quaternised nitrogen atom, the reaction is generally effected in the presence of an acid scavenging agent for example a base such as triethylamine or calcium carbonate.

In process (D) above, the compound of formula (Ib) is advantageously reacted with a compound of the formula $R^5W$ wherein $R^5$ is a $C_{1-4}$ alkyl group or a group of formula $H_2NCOCH_2-$, and W is a leaving group such as a halogen atom (e.g. iodine, chlorine or bromine) or a hydrocarbylsulphonate (e.g. mesylate or tosylate) group. Alternatively, a di-$C_{1-4}$-alkyl sulphate, e.g. dimethyl sulphate, may be employed as an alkylating agent. Iodomethane is preferred as the alkylating agent and iodoacetamide is preferred as the carbamoylmethylating agent. The reaction is preferably carried out at a temperature in the range of 0 to 60°C, advantageously 20 to 30°C. Where the alkylating agent is liquid under the reaction conditions, as in the case of iodomethane, this agent can itself serve as a solvent. Alternatively, the reaction may be conveniently effected in an inert solvent such as an ether e.g. tetrahydrofuran, an amide, e.g. dimethylformamide, a lower alkanol, e.g. ethanol, a lower dialkylketone, e.g. acetone, a halogenated hydrocarbon e.g. dichloromethane or an ester e.g. ethyl acetate.

The compound of formula (Ib) used as starting material in process (D) is a compound according the the present invention and may be prepared for example by reaction of a compound of formula (V) (as defined above) with an appropriate sulphur nucleophile in an analogous manner to the nucleophilic displacement reaction described with respect to process (C). If desired the above nucleophile may be used in the form of a metal thiolate salt.

When X in formula (V) is halogen, the reaction is preferably effected in the presence of an acid scavenging agent, for example a base such as triethylamine or calcium carbonate.

The reaction product may be separated from the reaction mixture, which may contain, for example, unchanged cephalosporin starting material and other substances, by a variety of processes including recrystallisation, ionophoresis, column chromatography and use of ion-exchangers (for example by chromatography on ion-exchange resins) or macroreticular resins.

A $\Delta^2$-cephalosporin ester derivative obtained in accordance with the process of the invention may be converted into the corresponding $\Delta^3$-derivative by, for example, treatment of the $\Delta^2$-ester with a base, such as pyridine or triethylamine.

Where a compound is obtained in which B is $>S\rightarrow O$ this may be converted into the corresponding sulphide by, for example, reduction of the corresponding acyloxysulphonium or alkoxysulphonium salt prepared in situ by reaction with e.g. acetyl chloride in the case of an acetoxysulphonium salt, reduction being effected by, for example, sodium dithionite or by iodide ion as in a solution of potassium iodide in a water-miscible solvent e.g. acetic acid, acetone, tetrahydrofuran, dioxan, dimethylformamide or dimethylacetamide. The reaction may be effected at a temperature of from -20° to +50°C.

Metabolically labile ester derivatives of the compounds of formula (I) may be prepared by reacting a compound of formula (I) or a salt or protected derivative thereof with the appropriate esterifying agent such as an acyloxyalkyl halide or alkoxycarbonyloxyalkyl halide (e.g. iodide) conveniently in an inert organic solvent such as dimethylformamide or acetone, followed, where necessary, by removal of any protecting groups.

Base salts of the compounds of formula (I) may be formed by reacting an acid of formula (I) with an appropriate base. Thus, for example, sodium or potassium salts may be prepared using the respective 2-ethylhexanoate or hydrogen carbonate salt. Acid

addition salts may be prepared by reacting a compound of formula (I) or a metabolically labile ester derivative thereof with the appropriate acid.

Conversion of a compound in which Y is a carboxyl group into a compound in which Y is an esterified carboxyl group or a carbamoyl or substituted carbamoyl group, may conveniently be effect *via* the formation of an activated intermediate, for example, a mixed anhydride formed with an alkylhalo formate. Such an activated intermediate may conveniently be formed *in situ*. Amination to form a compound in which Y is a carbamoyl or substituted carbamoyl group may be effected analogously to the process described in European Patent Application No. 95329.

Dehydration of a compound in which Y is a carbamoyl group to form a compound in which Y is a cyano group may be effected by conventional methods, e.g. using $PCl_5$ or $P_2O_5$.

Such modifications to the group Y will preferably be carried out using a compound in which $R^2$ represents a carboxyl blocking group.

Where a compound of formula (I) is obtained as a mixture of isomers, the *syn* isomer may be obtained by, for example, conventional methods such as crystallisation or chromatography.

Mixtures of enantiomers, including racemic mixtures, of compounds according to the invention or intermediates therefor may be resolved using conventional means, see for example "Stereochemistry of Carbon Compounds" by E.L. Elial (McGraw Hill, 1962) and "Tables of Resolving Agents" by S.H. Wilen.

For use as starting materials for the preparation of compounds of general formula (I) according to the invention, compounds of general formula (III) and the amide forming derivatives thereof such as acid halides and anhydrides corresponding thereto in their *syn* isomeric form or in the form of mixtures of the *syn* isomers and the corresponding *anti* isomers

containing at least 90% of the syn isomer are preferably used.

Acids of formula (III) and their derivatives are themselves novel compounds and form a further feature of the invention. They may be prepared by etherification of a compound of formula (VI)

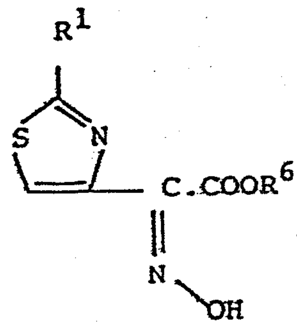

(VI)

(wherein $R^1$ is as hereinbefore defined and $R^6$ represents hydrogen or a carboxyl blocking group) or a salt thereof, by selective reaction with a compound of general formula (VII)

$T.Q.Y^b$ (VII)

(wherein Q and $Y^b$ are as defined above and T is a halogen atom, such as a chlorine, bromine or iodine atom; a sulphate group; or a sulphonate group, such as p-toluenesulphonate), followed by removal of any carboxyl blocking group $R^6$ and, if desired, removal of any carboxyl blocking group in $Y^b$. Separation of isomers may be effected either before or after such etherification. The etherification reaction is conveniently carried out in the presence of a base, e.g. potassium carbonate or sodium hydride, and is preferably conducted in an organic solvent, for example dimethylsulphoxide, a cyclic ether such as tetrahydrofuran or dioxan, or an N,N-disubstituted amide such as dimethylformamide. Under these conditions the configuration of the oxyimino group is substantially unchanged by the etherification reaction. The reaction should be effected in the presence of a base if an acid

addition salt of a compound of formula (VI) is used. The base should be used in sufficient quantity to neutralise rapidly the acid in question.

Acids of formula (III) may also be prepared by reaction of a compound of formula (VIII)

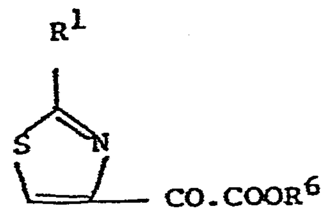

(VIII)

(wherein $R^1$ and $R^6$ are as hereinbefore defined) with a compound of formula (IX)

$$H_2N.O.Q.Y^b \qquad (IX)$$

(wherein Q and $Y^b$ are as hereinbefore defined) followed by removal of any carboxyl blocking group $R^6$, and, if desired, removal of any carboxyl blocking group in $Y^b$. Where necessary the reaction is also followed by the separation of syn and anti isomers.

The acids of formula (III) may be converted into the corresponding acid halides and anhydrides and acid addition salts by conventional methods, for example as described hereinabove.

Where X is a halogen atom in formula (V), ceph-3-em starting compounds may be prepared in conventional manner, e.g. by halogenation of a 7β-protected amino-3-methylceph-3-em-4-carboxylic acid ester 1β-oxide, removal of the 7β-protecting group, acylation of the resulting 7β-amino compound to form the desired 7β-acylamido group, e.g. in an analogous manner to process (A) above, followed by reduction of the 1β-oxide group later in the sequence. This is described in British Patent

Specification No. 1326531. The corresponding ceph-2-em compounds may be prepared by the method of Dutch published Patent Application No. 6902013 by reaction of a 3-methylceph-2-em compound with N-bromosuccinimide to yield the corresponding 3-bromomethylceph-2-em-compound.

Where X in formula (V) is an acetoxy group, such starting materials may be prepared for example by N-acylation of 7-aminocephalosporanic acid, e.g. in an analogous manner to process (A) above. Compounds of formula (V) in which X represents other acyloxy groups can be prepared by acylation of the corresponding 3-hydroxymethyl compounds which may be prepared for example by hydrolysis of the appropriate 3-acetoxymethyl compounds, e.g. as described for example in British Patent Specifications Nos. 1474519 and 1531212.

Compounds of formula (II) may also be prepared in conventional manner, e.g. by nucleophilic displacement of a corresponding 3-acyloxymethyl or 3-halomethyl compound with the appropriate nucleophile, e.g. as described in British Patent Specifications Nos. 1012943 and 1241657, and in British Patent Applications Nos. 2027691A, 2046261A and 2098216A.

A further method for the preparation of the starting materials of formula (II) comprises deprotecting a corresponding protected 7β-amino compound, for example in conventional manner, e.g. using $PCl_5$.

A further reference to the preparation of compounds of formula (II) is U.S. Patent No. 4145538.

It should be appreciated that in some of the above transformations it may be necessary to protect any sensitive groups in the molecule of the compound in question to avoid undesirable side reaction. For example, during any of the reaction sequences referred to above it may be necessary to protect the $NH_2$ group of the aminothiazolyl moiety, for example by tritylation, acylation (e.g.

chloroacetylation or formylation), protonation or other conventional method, so that the protected group is stable to the reaction conditions accompanying one or more synthetic steps. The protecting group may thereafter be removed in any convenient way which does not cause breakdown of the desired compound, e.g. in the case of a trityl group by using an optionally halogenated carboxylic acid, e.g. acetic acid, formic acid, chloroacetic acid or trifluoroacetic acid or using a mineral acid, e.g. hydrochloric acid or mixtures of such acids, preferably in the presence of a protic solvent such as water, or, in the case of a chloroacetyl group, by treatment with thiourea.

Carboxyl blocking groups used in the preparation of compounds of formula (I) or in the preparation of necessary starting materials are desirably groups which may readily be split off at a suitable stage in the reaction sequence, conveniently at the last stage. It may, however, be convenient in some instances to employ non-toxic metabolically labile carboxyl blocking groups such as acyloxy-methyl or -ethyl groups (e.g. acetoxy-methyl or -ethyl or pivaloyloxymethyl) or alkoxycarbonyloxyalkyl groups (e.g. ethoxycarbonyloxyethyl) and retain these in the final product to give an appropriate ester derivative of a compound of formula (I).

Suitable carboxyl blocking groups are well known in the art, a list of representative blocked carboxyl groups being included in British Patent No. 1399086. Preferred blocked carboxyl groups include aryl lower alkoxycarbonyl groups such as _p_-methoxybenzyloxycarbonyl, p-nitrobenzyloxycarbonyl and diphenylmethoxycarbonyl; lower alkoxycarbonyl groups such as _t_-butoxycarbonyl; and lower haloalkoxy-carbonyl groups such as 2,2,2-trichloroethoxycarbonyl. The carboxyl blocking group may subsequently be removed by any of the appropriate methods disclosed in the literature; thus, for example, acid or base

catalysed hydrolysis is applicable in many cases, as are enzymically-catalysed hydrolyses.

It will be appreciated that the use of amino protecting and carboxyl blocking groups is well known in the art and relevant examples of such use are given in e.g. Theodora W. Greene, "Protective Groups in Organic Synthesis" (Wiley-Interscience, New York, 1981), and J.F.W. McOmie, "Protective Groups in Organic Chemistry" (Plenum Press, London, 1973).

The antibiotic compounds of the invention may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds according to the invention may be formulated for injection and may be presented in unit dose form, in ampoules, or in multi-dose containers, if necessary with an added preservative. The compositions may also take such forms as suspensions, solutions, or emulsions in oily or aqueous vehicles, and may contain formulatory agents such as suspending, stabilising and/or dispersing agents. Alternatively the active ingredient may be in powder form for reconstitution with a suitable vehicle, e.g. sterile, pyrogen-free water, before use.

If desired, such powder formulations may contain an appropriate non-toxic base in order to improve the water-solubility of the active ingredient and/or to ensure that when the powder is reconstituted with water, the pH of the resulting aqueous formulation is physiologically acceptable. Alternatively the base may be present in the water with which the

powder is reconstituted. The base may be, for example, an inorganic base such as sodium carbonate, sodium bicarbonate or sodium acetate, or an organic base such as lysine or lysine acetate.

The antibiotic compounds may also for example be presented in a form suitable for absorption by the gastro-intestinal tract e.g. as tablets or capsules.

The antibiotic compounds may also, for example, be formulated as suppositories e.g. containing conventional suppository bases such as cocoa butter or other glycerides.

Compositions for veterinary medicine may, for example, be formulated as intramammary preparations in either long acting or quick-release bases.

The compositions may contain from 0.1% upwards, e.g. 0.1-99% of the active material, depending on the method of administration. When the compositions comprise dosage units, each unit will preferably contain 100-3000 mg e.g. 200-2000 mg of the active ingredient. The dosage as employed for adult human treatment will preferably range from 200 to 12000 mg e.g. 1000-9000 mg per day, depending on the route and frequency of administration. For example, in adult human treatment 400 to 4000 mg per day administered intravenously or intramuscularly will normally suffice. In treating *Pseudomonas* infections higher daily doses may be required. It will be appreciated that in some circumstances, for example in the treatment of neonates, smaller dosage units and daily dosages may be desirable.

The antibiotic compounds according to the invention may be administered in combination with other therapeutic agents such as antibiotics, for example penicillins or other cephalosporins.

The following Examples illustrate the invention. All temperatures are in °C. DMSO is dimethylsulphoxide.

Preparation 1

1-Hydroxymethyl-1-cyclopropylcarbonitrile

Methyl 1-cyano-1-cyclopropylcarboxylate (5.00g) in 1,2-dimethoxyethane (40ml) was treated with lithium borohydride (1.74g) and the mixture heated under reflux with stirring.

After 3h the suspension was allowed to cool to room temperature and then filtered. The solid was washed with 1,2-dimethoxyethane and the combined washings and filtrate evaporated. The residue was purified by chromatography on silica gel eluting with a mixture of ether and petrol (b.p.40-60°) (3:1) to give the title compound as a colourless oil (1.09g). Further elution with ether gave more product as a colourless oil (1.14g); $\nu_{max}$ ($CHBr_3$) 3595 (free OH), 3460 (bonded OH), and 2235 $cm^{-1}$ (CN).

Preparation 2

1-Bromomethyl-1-cyclopropylcarbonitrile

Phosphorus tribromide (0.70ml) was added dropwise to a stirred soluton of the product of Preparation 1 (1.92g) in ether (50ml) at -78° under nitrogen.

After 5h at -78° the cooling bath was removed and the reaction allowed to stand for 15h. Water (10ml) was added and the mixture shaken. The organic phase was separated off, washed with saturated sodium bicarbonate solution, dried over magnesium sulphate and evaporated. The residue was purified by chromatography on silica gel (100g) eluting with a mixture of ether and petrol (b.p. 40-60°) (1:1) to give the title compound as a colourless oil (1.60g), $\nu_{max}$ ($CHBr_3$) 2230 $cm^{-1}$ (CN).

Preparation 3

1-Bromomethyl-1-cyclopropanecarboxamide

The product of Preparation 2 (1.5g) was dissolved

in 98% sulphuric acid (6ml) and heated to 50° for 2.3h.

The solution was added dropwise to rapidly stirred, cooled (5°) water (20ml). This solution was extracted with ether (x8) and the extracts combined, dried over magnesium sulphate and evaporated. The residue was purified by chromatography on silica gel (15g) eluting first with ether and then a mixture of dichloromethane and ether (1:19) to give the title compound as crystals (0.44g); $\nu_{max}$ ($CHBr_3$) 3498 and 3395 ($-NH_2$), and 1673 $cm^{-1}$ (C=0).

Preparation 4

(a) 4-Nitrobenzyl (Z)-2-(1-diphenylmethoxycarbonyl-cyclopropylmethoxyimino)-2-(2-tritylaminothiazol-4-yl)acetate

A mixture of 4-nitrobenzyl (Z)-2-(hydroxyimino)-2-(2-tritylaminothiazol-4-yl)acetate (1.00g), potassium carbonate (0.95g) and diphenylmethyl 1-bromomethyl-1-cyclopropylcarboxylate (prepared according to the method described in British Patent No. 1,496,757) (0.70g) in DMSO (5ml) was stirred under nitrogen at ambient temperature. After 3h more diphenylmethyl 1-bromomethyl-1-cyclopropyl carboxylate (0.14g) was added and the reaction stirred for a further 1h. The suspension was added dropwise to rapidly stirred water (75ml). The precipitated solid was filtered off, washed with water and dried in vacuo. The solid was purified by chromatography on silica gel (50g) eluting with a mixture of ethyl acetate and petrol (b.p. 40-60°) (1:3) to give the title compound as a foam (0.91g); $\nu_{max}$ ($CHBr_3$) 3399 (NH), 1730 (C=0), and 1525 and 1349 $cm^{-1}$ ($NO_2$).

(b) (Z)-2-(1-Diphenylmethoxycarbonylcyclopropylmeth-oxyimino)-2-(2-tritylaminothiazol-4-yl)acetic acid

Sodium dithionite (1.08g) was added to a stirred two-phase solution of the product of Preparation

4(a) (0.64g) in tetrahydrofuran (35ml), saturated sodium bicarbonate solution (11ml), sodium carbonate (1.8g), water (23ml) and ice (11g). After 2h the mixture was partitioned between water (150ml) and ethyl acetate (100ml) and acidified using 2N hydrochloric acid. The organic phase was separated off, washed with water (2 x 150ml), dried over sodium sulphate and evaporated to give the title compound as a yellow foam (0.66g); $\nu_{max}$ (Nujol) 3600-3300 ($NH,H_2O$), and 1775 $cm^{-1}$ (C=0,ester); and $\tau$ ($DMSO-d_6$) 3.42 (thiazolyl), 5.64 ($OC\underline{H}_2$), and 8.65-9.0 (cyclopropyl).

Preparation 5

(a) 4-Nitrobenzyl (Z)-2-(1-cyanocyclopropylmethoxyimino)-2-(2-tritylaminothiazol-4-yl)acetate

The title compound was prepared according to the method of Preparation 4(a) and exhibited $\nu_{max}$ ($CHBr_3$) 3395 (NH), 2235 (CN), 1740 (C=0), and 1522 and 1345 $cm^{-1}$ ($NO_2$).

(b) (Z)-2-(1-cyanocyclopropylmethoxyimino)-2-(2-tritylaminothiazol-4-yl)acetic acid

The title compound was prepared according to the method of Preparation 4(b) and exhibited $\nu_{max}$ (Nujol) 3360 ($NH,H_2O$) and 2235 $cm^{-1}$ (CN); and $\tau$($DMSO-d_6$) 3.32 (thiazolyl), 6.08 ($OC\underline{H}_2$), and 8.68-8.93 (cyclopropyl).

Preparation 6

a) 4-Nitrobenzyl (Z)-2-(1-carbamoylcyclopropylmethoxyimino)-2-(2-tritylaminothiazol-4-yl)acetate

The title compound was prepared according to the method of Preparation 4(a) and exhibited $\nu_{max}$ (Nujol), 3475 (NH), 3350-3100 ($NH_2$), 1750 (C=0,ester), 1658 (C=0, amide) and 1525 and 1348 $cm^{-1}$ ($NO_2$).

(b) (Z)-2-(1-carbamoylcyclopropylmethoxyimino)--2-(tritylaminothiazol-4-yl)acetic acid

The title compound was prepared according to the method of Preparation 4(b) and exhibited $\nu_{max}$ (Nujol) 1710 (C=O,acid), 1660 (C=O,amide); and τ (DMSO-$d_6$) 5.8 ($OCH_2$) and 8.9-9.3 (cyclopropyl).

Preparation 7

Diphenylmethyl cyclopropylglyoxalate

A stirred solution of cyclopropylglyoxylic acid (prepared according to the method of I. Basnak and J. Farkas, Coll.Czech. Chem.Comm. 40, 1038 (1975))

(1.83g) in dichloromethane (20ml) was treated dropwise with a solution of diphenyldiazomethane in dichloromethane (0.5M) until a permanent pink colour was obtained. After 1 hour the mixture was allowed to stand overnight and then evaporated. The residue was purified on a column of silica gel (120g) eluting with a mixture of diethyl ether and petrol (1:5) to give the title compound as a liquid (3.38g); $\nu_{max}$ ($CHBr_3$) 1728 (C=O,ester) and 1710 $cm^{-1}$ (C=O,ketone); and τ($CDCl_3$) 3.05 ($-CHPh_2$), 7.29 and 8.65-9.00 (cyclopropyl ring).

Preparation 8

Diphenylmethyl α-hydroxycyclopropylacetate

Sodium borohydride (0.22g) was added to a stirred solution of the product of Preparation 7 (1.65g) in 1,2-dimethoxy ethane (15ml) at -20°. After 7 min the mixture was poured into a stirred mixture of dichloromethane (100ml), water (75ml) and 1M hydrochloric acid (24ml). After a few minutes the two layers were separated and the aqueous phase was extracted with dichloromethane (2 x 50ml). The combined organic extracts were washed with saturated sodium bicarbonate solution (100ml) and water (100ml), dried over sodium sulphate, and

evaporated. The residue was purified on a column of silica gel (40g), eluting with a mixture of diethyl ether and petrol (1:1) to give the title compound as a liquid (1.27g); $\lambda_{max}$ (ethanol) 258.5nm ($\varepsilon$ 650) and $\tau$ ($CDCl_3$) 6.18 (C<u>H</u>-OH), 7.24 (-O<u>H</u>), 8.88 and 9.25-9.75 (cyclopropyl ring).

Preparation 9

Diphenylmethyl α-mesyloxycyclopropylacetate

A solution of mesyl chloride (0.36ml) in dichloromethane (5ml) was added dropwise, over 15 mins, to a stirred solution of the product of Preparation 8 (1.20g) and triethylamine (0.89ml) in dichloromethane (12ml) at -10°. After stirring for a further 30 min the mixture was diluted with dichloromethane (90ml) and washed with saturated sodium bicarbonate solution (100ml) and saturated sodium chloride solution (100ml), dried over sodium sulphate and evaporated to give the title compound as a low-melting solid (1.50g); $\nu_{max}$ ($CHBr_3$) 1750 (C=O) and 1354$cm^{-1}$ ($-OSO_2$); and $\tau$ ($CDCl_3$) 5.54 ($-C\underline{H}CO_2-$), 7.01 ($-OSO_2CH_3$), 8.30-8.90 and 9.10-9.50 (cyclopropyl ring).

Preparation 10

4-Nitrobenzyl (Z)-2-(α-diphenylmethoxycarbonylcyclopropyl methoxyimino)-2-(2-tritylaminothiazol-4-yl)acetate

A mixture of the product of Preparation 9 (100mg), potassium carbonate (237mg) and 4-nitrobenzyl (Z)-2-(hydroxyimino)-2-(2-tritylaminothiazol-4-yl)acetate (237mg) in DMSO (2ml) was stirred for 21.25 h under nitrogen. The mixture was added to vigorously stirred water (50ml) and the precipitated solid was extracted with ethyl acetate (50ml and 2 x 25ml). The combined organic extracts were washed with water (2 x 50ml) and saturated sodium chloride solution (50ml), dried over sodium sulphate

and evaporated. The residue was purified on a column of silica gel (15g), eluting with a mixture of ethyl acetate and petrol (1:3) to give the title compound as a foam (189mg); $\lambda_{max}$ (ethanol) 250nm ($\varepsilon$ 23300); and $\tau$ ($CDCl_3$) 1.94 and 2.46 ($-C_6\underline{H}_4NO_2$), 3.10 ($-C\underline{H}Ph_2$) 3.18 (NH), 3.51 (thiazole 5$\underline{H}$), 4.60 ($OC\underline{H}_2-$), 5.66 (OC$\underline{H}$-cyclopropyl), 8.60-9.00 and 9.25-9.60 (cyclopropyl ring).

Preparation 11

(Z)-2-(α-diphenylmethoxycarbonylcyclopropylmethoxy-imino)-2-(2-tritylaminothiazol-4-yl)acetic acid

Sodium dithionite (1.91g) was added to a stirred mixture of the product of Preparation 10 (1.91g) in tetrahydrofuran (75ml), saturated sodium bicarbonate solution (25ml), sodium carbonate (1.5g), water (50ml) and ice (ca 25g). After 40 min the mixture was poured into a stirred mixture of ethyl acetate (150ml) and water (150ml) and dilute hydrochloric acid was added until pH 1. The two layers were separated and the aqueous phase was extracted with ethyl acetate (50ml). The combined organic extracts were washed with water (150ml), dilute hydrochloric acid (150ml) water (150ml) and saturated sodium chloride solution (150ml), dried over sodium sulphate and evaporated. The residue was triturated with diethyl ether and the solid was collected by filtration, washed with more ether and dried in vacuo to give the title compound as a solid (0.897); $\nu_{max}$ (Nujol) 1745 (C=O,ester) and 1715 $cm^{-1}$ (C=O,acid); and $\tau$ (DMSO-$d_6$) 1.14 (NH), 3.13 ($-C\underline{H}Ph_2$) 3.21 (thiazole-5$\underline{H}$), 5.81 (OC$\underline{H}$-cyclopropyl), 8.82 and 9.20-9.70 (cyclopropyl ring).

Preparation 12

1-Bromomethylcyclopropanecarboxylic acid, methyl ester

1-Bromomethylcyclopropanecarboxylic acid (2.06g) in methanol (10ml) was treated with 98% sulphuric acid (0.5ml) and the solution heated at reflux for 17h. The mixture was evaporated to dryness and the residue partitioned between ether (100ml) and water (50ml). The organic phase was separated off and washed successively with saturated aqueous sodium bicarbonate (50ml), water (50ml) and then brine (50ml). The solvent was evaporated and the residue purified by chromatography over silica gel (100g), eluting with ether/petrol (1:4) to give the title ester as a colourless oil (0.73g); $\nu_{max}$ ($CHBr_3$) 1728 $cm^{-1}$ (ester C=O).

Preparation 13

4-Nitrobenzyl (Z)-2-(1-methoxycarbonylcyclopropylmethoxyimino)-2-(2-tritylaminothiazol-4-yl)acetate

The title compound was prepared according to the method of Preparation 4(a) from the product of Preparation 12 and exhibited $\nu_{max}$ ($CHBr_3$) 3400 (NH), 1730 (ester C=O) 1525, 1348 $cm^{-1}$ ($NO_2$).

Preparation 14

(Z)-2-(1-methoxycarbonylcyclopropylmethoxyimino)-2-(2-tritylaminothiazol-4-yl)acetic acid

The title compound was prepared according to the method of Preparation 4(b) from the product of Preparation 13 and exhibited $\nu_{max}$ (Nujol) 1720 $cm^{-1}$ (C=O), and $\tau$ (DMSO-$d_6$) 5.76 ($OCH_2$), 8.6-9.2 (cyclopropyl).

Preparation 15

α-Oxocyclopropaneacetic acid, methyl ester

A mixture of α-oxocyclopropaneacetic acid, potassium salt (1.00g) and methyl iodide (0.82ml) in dimethylsulphoxide (5ml) was stirred for 1.75 hours. The resulting pale yellow solution was passed into water (100ml) and extracted with ether (3 x 50ml). The combined extracts were washed with water (2 x 50ml) and brine (50ml) and dried (magnesium sulphate). The solvent was removed to give the title compound as a colourless liquid (0.669g); $\nu_{max}$ ($CHBr_3$) 1730 (ester C=O), 1710 $cm^{-1}$ (Ketone C=O).

Preparation 16

α-Hydroxycyclopropaneacetic acid, methyl ester

Sodium borohydride (1.08g) was added to a stirred solution of the product of Preparation 15 (3.66g) in methanol (35ml) at -20°. After 5 minutes the mixture was cautiously poured into a stirred mixture of dichloromethane (250ml) water (85ml) and 1M hydrochloric acid (115ml). The two layers were separated and the aqueous phase was extracted with dichloromethane (3 x 100ml). The combined organic extracts were washed with saturated sodium bicarbonate solution (100ml) and water (100ml) and dried (sodium sulphate). The solvent was removed to give the title compound as a colourless liquid (2.63g); $\nu_{max}$ ($CHBr_3$) 3535 (OH), 1732 $cm^{-1}$ (C=O).

Preparation 17

α-Methylsulphonyloxycyclopropaneacetic acid, methyl ester

A solution of methanesulphonyl chloride (1.66ml) in dichloromethane (20ml) was added dropwise, over 20 minutes, to a stirred solution of the product of Preparation 16 (2.53g) and triethylamine (4.06ml) in dichloromethane (40ml) at -10°. After 10 minutes the mixture was diluted with dichloromethane (100ml) and washed with ice-water (150ml), saturated sodium bicarbonate solution (100ml) and brine (150ml)

and dried (sodium sulphate). The solvent was removed to give the title compound as a white crystalline solid (3.68g); mp. 109.5°; $\nu_{max}$ ($CHBr_3$); 1755 (C=O), 1355 $cm^{-1}$ ($OSO_2$).

Preparation 18

4-Nitrobenzyl (Z)-2-(α-methoxycarbonylcyclopropylmethoxyimino)-2-(2-tritylaminothiazol-4-yl)acetate

A mixture of the product of Preparation 17, (2.43g), potassium carbonate (6.0g) and 4-nitrobenzyl (Z)-2-(hydroxyimino)-2-(2-tritylaminothiazol-4-yl)acetate (6.00g) in DMSO (30ml) was stirred, under nitrogen, for 6.33h.

The mixture was partitioned between ethyl acetate (250ml) and water (250ml). The two layers were separated and the aqueous phase was extracted with ethyl acetate (2 x 100ml). The combined extracts were washed with water (250ml), 2M hydrochloric acid (250ml), water (250ml) and brine (250ml) and dried (sodium sulphate). The solvent was removed and the residue was purified by chromatography (150g silica gel). Elution with ethyl acetate: petrol (1:3) gave the title compound as a yellow foam (5.38g); $\lambda_{max}$ EtOH 239 nm (ε 21,500); $\nu_{max}$ ($CHBr_3$) 3385 (NH), 1748 (C=O), 1522 and 1348 $cm^{-1}$ ($NO_2$), and τ ($CDCl_3$) 3.37 (thiazole), 5.71 (O-CH), and 9.2-9.6 (cyclopropyl).

Preparation 19

(Z)-2-(α-Methoxycarbonylcyclopropylmethoxyimino)-2-(2-tritylaminothiazol-4-yl)acetic acid

Sodium dithionite (5.15g) was added to a stirred mixture of the product of Preparation 18 (5.15g) in tetrahydrofuran (125ml), saturated sodium bicarbonate solution (40ml), sodium carbonate (5.0g), water (75ml) and ice (ca 40g). After 90 minutes the

mixture was poured into a stirred mixture of ethyl acetate (250ml) and water (250ml) and 2M hydrochloric acid was added until the aqueous phase was acidic (pH 1). The two layers separated and the aqueous phase was extracted with EtOAc (125ml). The combined organic extracts were washed with water (250ml), 2M hydrochloric acid (250ml), water (250ml) and brine (250ml), and dried (sodium sulphate). The solvent was removed and the residue was triturated with ether. The solid was collected by filtration, washed with ether and dried _in vacuo_ to give the _title compound_ as a pale yellow, powdery solid (2.89 g); $\lambda_{inf}$ (EtOH) 233 nm ($\varepsilon$ 20,500), 259.5 nm ($\varepsilon$ 11,100), 265 nm ($\varepsilon$ 11,000); $\nu_{max}$ (Nujol) 3265 (NH), 1742 (C=O ester), 1715 (C=O acid) and $\tau$ (DMSO-$d_6$) 3.14 (thiazole), 5.94 (OCH), 9.3-9.65 (cyclopropyl).

_Example 1_

_(6R,7R)-7-[(Z)-2-(2-aminothiazol-4-yl)-2-(1-carboxy-cyclopropylmethoxyiminoacetamido]-3-pyridiniummethyl-ceph-3-em-4-carboxylate dihydrochloride_

Oxalyl chloride (0.16ml) was added to a stirred solution of dimethylformamide (0.16ml) in dichloromethane (4.3ml) at -20° under nitrogen. The mixture was stirred at 5° for 10 min and then recooled to -20°. The product of Preparation 4(b) (1.19g) was added and the resulting solution stirred at 5° for 10 min before recooling to -20°.

Meanwhile, in a separate flask, triethylamine (1.00ml) and then industrial methylated spirits (5.1ml) were added to a rapidly stirred solution of (6R,7R)-7-amino-3-pyridiniummethylceph-3-em-4-carboxylate dihydrochloride (0.65g) in water (1.3ml). The solution of activated side chain acid was added and the mixture allowed to warm to room temperature over 15 min. The suspension was twice washed with water, each time back-extracting

with dichloromethane. The organic phases were combined; dried over sodium sulphate and evaporated to a foam. This was dissolved in formic acid (4.89ml) and treated with concentrated hydrochloric acid (0.39ml). After 1h the mixture was filtered and the solid washed with formic acid. The combined washings and filtrate were evaporated and the residue triturated with acetone to give a solid. This was purified by chromatography over silica gel (30g) eluting with a mixture of acetone and water (9:1 to 4:1) to give the title compound as an off-white solid (0.07g); $\lambda_{max}$ (ethanol) 236nm (ε 17935); and $\nu_{max}$ (Nujol) 2700, and 1700 ($CO_2H$), 1776 (β-lactam), and 1670 and 1540 $cm^{-1}$ (-CONH-).

Example 2

(6R,7R)-7-[(Z)-2-(2-Aminothiazol-4-yl)-2-(1-cyano-cyclopropylmethoxyiminoacetamido]-3-pyridiniummethylceph-3-em-4-carboxylate dihydrochloride

The title compound was prepared according to the method of Example 1 using the product of Preparation 5(b) as starting material. The title compound exhibited $\nu_{max}$ (Nujol), 2238 (CN), 1785 (β-lactam), 1705 (C=O,acid) and 1678 $cm^{-1}$ (C=O,amide); and τ (DMSO-$d_6$), 0.88, 1.30 and 1.75 (pyridinium), 3.07 (thiazolyl), 5.84 ($OCH_2$) and 8.6-8.9(cyclopropyl).

Example 3

(a) Diphenylmethyl(6R,7R)-7-[(Z)-2-(1-diphenylmethoxy-carbonylcyclopropylmethoxyimino)-2-(2-tritylaminothiazol-4-yl)acetamido]-3-carbamoyloxymethylceph-3-em-4-carboxylate

A solution of oxalyl chloride (0.11ml) in dichloromethane (1.5ml) was added dropwise over 5 min to a solution of dimethylformamide (0.14ml) in dichloromethane (6ml) at -115° under nitrogen and with stirring. This solution was stirred at -5° when a white precipitate formed. This mixture

was cooled to -10° and the product of Preparation 4(b) (0.81g) was added in one portion. The resulting clear brown solution was stirred at -5° for 30 min before recooling to -10°.

Meanwhile, in a separate flask, N,N-dimethylaniline (0.30ml) was added to a solution of diphenylmethyl (6R,7R)-7-amino-3-carbamoyloxymethylceph-3-em-4-carboxylate (0.51g) in dichloromethane (8ml) at -25°. The solution of active side chain was poured in and the mixture allowed to warm to 10° over 2h. Water (50ml) was added and the layers shaken. The organic layer was separated and washed with 2N hydrochloric acid (2 x 50ml), water (50ml), saturated sodium bicarbonate solution (50ml) and saturated sodium chloride solution (50ml). The organic phase was dried over sodium sulphate and evaporated. The residue was purified by chromatography on silica gel (50g), eluting with a mixture of ehtyl acetate and petrol (b.p. 40-60°) 1:6) to give the <u>title compound</u> as a pale yellow foam (0.59g); $\lambda_{max}$ (ethanol) 263nm (ε 19400); $\nu_{max}$ ($CHBr_3$) 3535 and 3408 ($NH_2$,NH), 1790 (β-lactam), and 1687 $cm^{-1}$ (amide).

<u>b) (6R,7R)-7-[(Z)-2-(2-aminothiazol-4-yl)-2-(1-carboxycyclopropylmethoxyimino)acetamido]-3-carbamoyloxymethylceph-3-em-4-carboxylate</u>

The product of Example 3(a) (0.44g) in anisole (0.89ml) was treated with trifluoracetic acid (3.82ml) and the mixture stirred for 30 min. the solution was added dropwise to rapidly stirred water (300ml). the resultant murky solution was washed with ether (3 x 100ml), partially evaporated, and finally freeze-dried to give the <u>title compound</u> was a white solid (0.23g); $\lambda_{max}$ (ethanol) 237nm (ε 19200); and $\nu_{max}$ (Nujol) 2600 and 1720 ($-CO_2H$), 1772 (β-lactam), and 1688 and 1542 $cm^{-1}$ (-CONH-).

Example 4

a) Diphenylmethyl (6R,7R)-7-[(Z)-2-(1-cyanocyclopropylmethoxyimino)-2-(2-tritylaminothiazol-4-yl)acetamido]-3-carbamoyloxymethylceph-3-em-4-carboxylate

The title compound was prepared according to the method of Example 3(a) using the product of Preparation 5(b) as starting material. The title compound exhibited $\nu_{max}$ ($CHBr_3$) 3535 and 3405 ($NH_2$,NH), 2235 (CN) and 1788 $cm^{-1}$ (β-lactam); and τ ($CDCl_3$) 3.02 (NH), 3.04 ($-CO_2CHPh_2$), 3.22 (thiazolyl), 4.96 and 5.22 ($-C\underline{H}_2$-OCO-), 5.74 ($-OCH_2-$) and 8.6-9.0 (cyclopropyl).

b) (6R,7R)-7-[(Z)-2-(2-aminothiazol-4-yl)-2-(1-cyanocyclopropylmethoxyimino)acetamido]-3-carbamoyloxymethylceph-3-em-4-carboxylate

The title compound was prepared according to the method of Example 3(b) using the product of stage a) and exhibited $\lambda_{max}$ (ethanol) 235nm (ε 19600); and $\nu_{max}$ (Nujol), 3300 (NH), 2240 (CN), 1772 (β-lactam), and 1665 and 1548 $cm^{-1}$ (-CONH).

Example 5

a) Diphenylmethyl (6R,7R)-3-carbamoyloxymethyl-7-[(Z)-2-(α-diphenylmethoxycarbonylcyclopropylmethoxyimino)-2-(2-tritylaminothiazol-4-yl)acetamido]ceph-3-em-4-carboxylate

A solution of oxalyl chloride (67ul) in dichloromethane (2ml) was added dropwise to a stirred solution of dimethylformamide (0.18ml) in dichloromethane (4ml), under nitrogen at -15°. The resulting suspension was stirred at ca -5° for 30 min and re-cooled to -10°. the product of Preparation 11 (500mg) was added and the resulting solution was stirred at ca -5° for 30 min before being re-cooled to -20°.

The solution of activated side chain was added to a stirred solution of diphenylmethyl (6R,7R)-7-amino-3-carbamoyloxymethylceph-3-em-4-carboxylate (308mg) and N,N-dimethylaniline (0.20ml) in dichloromethane (8ml), under nitrogen, at -25°. The solution was allowed to warm to +5° over 1h, diluted with dichloromethane (50ml) and poured into water (100ml). The two layers were separated and the organic phase was washed with dilute hydrochloric acid (100ml), water (100ml), saturated sodium bicarbonate solution (100ml) and saturated sodium chloride solution (100ml), dried over sodium sulphate and evaporated. The residue was purified on a column of siliça gel (30g), eluting with a mixture of ethyl acetate and dichloromethane (1:9) to give the title compound as a foam (440mg); $\nu_{max}$ ($CHBr_3$) 3535, 3415 and 3275 ($NH,NH_2$), 1790 (C=0, β-lactam), 1729 (C=0, ester and $-OCONH_2$) and 1680 $cm^{-1}$ (C=0, amide); and τ ($CDCl_3$) 1.86 and 1.91 (CONH) 3.22 (thiazole -5H), 3.98 and 4.04 (7-H), 4.89, 4.91, 5.18 and 5.23 ($C\underline{H}_2OCONH_2$), 5.01 and 5.02 (6-H), 5.44 ($OCONH_2$), 5.52 and 5.62 (OCH-cyclopropyl), 6.52, 6.63, 6.72 and 6.90 ($2-C\underline{H}_2$), 8.50-8.80 and 9.20-9.50 (cyclopropyl ring.)

b) (6R,7R)-7-[(Z)-2-(2-Aminothiazol-4-yl)-2-(α-carboxycyclopropylmethoxyimino)acetamido]-3-carbamoyloxymethylceph-3-em-4-carboxylic acid, trifluoroacetate salt

The product of Example 5(a) (411mg) was stirred in trifluoroacetic acid (4ml) and anisole (1ml) for 1h. The solution was added dropwise to vigorously stirred water (50ml). Diethyl ether (50ml) was added and after a few minutes the two layers were separated. The organic phase was extracted with water (2 x 25ml) and the combined aqueous extracts were washed with diethyl ether (3 x 25ml), back washing each time with water (10ml). The aqueous

solution was concentrated and freeze-dried to give the title compound as a foam (229mg); $\lambda_{max}$ (ethanol) 236nm (ε 17300); and τ (DMSO-$d_6$) 0.55 (-CONH-), 3.14 and 3.16 (thiazole 5-H), 3.41 (-$OCONH_2$), 4.11 and 4.15 (7-H), 4.78 and 4.79 (6-H), 5.08 and 5.33 (-$C\underline{H}_2OCONH_2$), 5.99 and 6.03 (OC$\underline{H}$-cyclopropyl), 6.37 and 6.53 (2-$CH_2$) 8.60-8.90 and 9.25-9.60 (cyclopropyl ring).

Example 6

a) Diphenylmethyl (6R,7R)-7-[(Z)-2-(α-methoxycarbonylcyclopropylmethoxyimino)-2-(2-tritylaminothiazol-4-yl)acetamido]-3-carbamoyloxymethylceph-3-em-4-carboxylate

A solution of oxalyl chloride (0.19ml), in dichloromethane (2ml) was added dropwise to a stirred solution of dimethylformamide (0.34ml) in dichloromethane (3ml) at -15°, under nitrogen. The resulting white suspension was stirred at between -10° and -5° for 30 minutes and then cooled to -10°. The product of Preparation 19 (1.083g, 2.0 mmol) was added, washed in with a little dichloromethane, and the resulting green solution was stirred at ca -5° for 30 mins before being recooled to -20°.

The solution of activated side-chain acid was added in one portion to a stirred solution of diphenylmethyl (6R,7R)-7-amino-3-carbamoyloxymethylceph-3-em-4-carboxylate (0.879g) and N,N-dimethylaniline (0.56ml) in dichloromethane (15ml), under nitrogen, at -25°. The yellow solution was allowed to warm to +10° over 90 minutes, diluted with dichloromethane (50ml) and washed with water (100ml), 2M hydrochloric acid (100ml), water (100ml), saturated sodium bicarbonate solution (100ml) and brine (100ml) and dried (sodium sulphate). The solvent was removed and the residue was purified by chromatography (50g silica gel). Elution with ethyl acetate: dichloromethane (1:7) gave the title compound as a pale yellow foam (1.568

g); $\lambda_{inf}$ (EtOH) 236nm ($\epsilon$ 26,200) 258 nm ($\epsilon$ 19,600), 264 nm ($\epsilon$ 18,300); $\nu_{max}$ ($CHBr_3$), 3540, 3415, 3280, (NH, $NH_2$), 1792 $cm^{-1}$ ($\beta$-lactam C=O). $\tau$ ($CDCl_3$) 5.62, 5.72 (OCH, mixture of diastereoisomers) 9.22-9.5 (cyclopropyl).

b) (6R,7R)-7-[(Z)-2-(2-aminothiazol-4-yl)-2-($\alpha$-methoxycarbonylcyclopropylmethoxyimino)acetamido]-3-carbamoyloxymethylceph-3-em-4-carboxylic acid, trifluoroacetate salt

A stirred solution of the product of stage a) (1.00g) in anisole (2ml) was treated with trifluoroacetic acid (8ml). After 1 hour the solution was added to vigorously stirred water (100ml). Ether (100ml) was added and, after a few minutes, the two layers were separated. The organic phase was extracted with water (2 x 50ml) and the combined aqueous extracts were washed with ether (2 x 50ml), back-washing each time with water (10ml). The aqueous solution was concentrated by rotary evaporation and freeze-dried to give the title compound as a white foam (0.70g); $\lambda_{max}$ (EtOH) 235.5 nm ($\epsilon$ 16,500), $\nu_{max}$ (Nujol) 1782 ($\beta$-lactam C=O), 1725 (ester C=O) and $\tau$ (DMSO-$d_6$) 3.12, 3.14 (thiazole, mixture of diastereoisomers), 5.91 (OCH), 9.25-9.6 (cyclopropyl).

Example 7

a) Diphenylmethyl (6R,7R)-7-[(Z)-2-(1-carbamoylcyclopropylmethoxyimino)-2-(2-tritylaminothiazol-4-yl)acetamido]-3-carbamoyloxymethylceph-3-em-4-carboxylate

A mixture of the product of Preparation 6(b) (0.75g), diphenylmethyl (6R,7R)-7-amino-3-carbamoyloxymethylceph-3-em-4-carboxylate (0.75g), 1-hydroxy-1H-benzotriazole hydrate (0.19g), dicyclohexylcarbodiimide (0.29g) and tetrahydrofuran (12ml) was stirred for 20h. The mixture was filtered and the solid

was washed with tetrahydrofuran. The filtrate and washings were combined and evaporated and the residue partitioned between water (80ml) and dichloromethane (80ml). The organic phase was separated off and was washed successively with 2N hydrochloric acid (2 x 50ml), water (50ml), saturated aqueous sodium bicarbonate (50ml) and saturated brine (50ml). The organic phase was dried and then evaporated to give a brown foam. This was purified by chromatography over silica gel (300g) eluting first with ethyl acetate: petrol (4:1) and then ethyl acetate to give title compound as a yellow-brown foam (0.67g); $\nu_{max}$ ($CHBr_3$) 3510, 3400 (NH, $NH_2$), 1789 (β-lactam C=O), 1730 (ester urethane C=O), 1670 $cm^{-1}$ (amide C=O), and τ ($CDCl_3$) 5.71 ($OCH_2$), 8.6-9.3 (cyclopropyl).

b) (6R,7R)-7-[(Z)-2-(2-aminothiazol-4-yl)-2-(1-carbamoylcyclopropylmethoxyimino)acetamido]-3-carbamoyloxymethylceph-3-em-4-carboxylic acid, trifuoroacetate salt

The title compound was prepared from the product of stage a) according to the method of Example 3(b) and exhibited $\nu_{max}$ (Nujol) 1772 $cm^{-1}$ (β-lactam C=O), and τ(DMSO-$d_6$) 5.71 ($OCH_2$), 8.88-9.2 (cyclopropyl).

Example 8

a) Diphenylmethyl (6R,7R)-7-[(Z)-2-(1-methoxycarbonylcyclopropylmethoxyimino)-2-(2-tritylaminothiazol-4-yl)acetamido]-3-carbamoyloxymethylceph-3-em-4-carboxylate

The title compound was prepared according to the method of Example 7 a) from the product of Preparation 14 and exhibited $\nu_{max}$ ($CHBr_3$) 3540, 3410 (NH, $NH_2$), 1790 (β-lactam C=O), 1729 (ester,

urethene C=O), 1683 $cm^{-1}$ (amide C=O), and τ ($CDCl_3$) 5.55, 5.66 ($OCH_2$), 8.68-9.0 (cyclopropyl).

b) (6R,7R)-7-[(Z)-2-(2-aminothiazol-4-yl)-2-(1-methoxycarbonylcyclopropylmethoxyimino)acetamido]-3-carbamoyloxymethylceph-3-em-4-carboxylic acid, trifluoroacetate salt

The title compound was prepared from the product of stage a) according to the method of Example 3b) and exhibited $\nu_{max}$ (Nujol) 1772 (β-lactam C=O), 1721 (ester C=O), 1678 (amide C=O), and τ ($DMSO-d_6$) 5.68 ($OCH_2$), 8.7-9.0 (cyclopropyl).

Example 9

(6R,7R)-7-[(Z)-2-(2-aminothiazol-4-yl)-2-(1-methoxycarbonylcyclopropylmethoxyimino)acetamido]-3-pyridiniummethylceph-3-em-4-carboxylate dihydrochloride

The title compound was prepared from the product of Preparation 14 according to the method of Example 1 and exhibited $\nu_{max}$ (Nujol) 1788 (β-lactam C=O), 1718 (ester C=O), 1678 $cm^{-1}$ (amide C=O), and τ ($DMSO-d_6$) 5.6 - 5.8 ($OCH_2$), 8.65-9.0 (cyclopropyl), $\lambda_{max}$ (ethanol) 236.5 nm (ε 13,400).

Example 10

(6R,7R)-7-[(Z)-2-(2-aminothiazol-4-yl)-2-(1-carbamoylcyclopropylmethoxyimino)acetamido]-3-pyridiniummethylceph-3-em-4-carboxylate dihydrochloride

A mixture of the product of Preparation 6(b) (0.84g), 1-hydroxy-1H-benzotriazole hydrate (0.22g), dicyclohexylcarbodiimide (0.36g) and dimethylformamide (11ml) was stirred for 3h at room temperature and then filtered. The filtrate was added to a stirred and cooled (-40°) solution of (6R,7R)-7-amino-3-pyridiniummethylceph-3-em-4-carboxylate, dihydrochloride, dihydrate (0.70g) in dimethylformamide and dimethylaniline (0.65ml) and the mixture was

left overnight at room temperature. The suspension was filtered and the yellow brown filtrate was added dropwise to ether (400ml). the supernatant liquid was decanted off and the precipitate stirred together with acetone, filtered off, washed with acetone and dried by suction. The crude product was partitioned between water (50ml) and dichloromethane (50ml). The organic layer was separated off and extracted with water (3 x 50ml), each time back-extracting with a little dichloromethane. The organic solutions were combined and then washed with saturated brine and dried. The solvent was removed by evaporation to give a brown foam (1.08g).

This was dissolved in formic acid (4.89ml) and treated with concentrated hydrochloric acid (0.39ml) and the mixture stirred for 1h at room temperature. The suspension was filtered and the solid washed with formic acid. The filtrate and washings were combined and evaporated to a brown gum which was triturated with acetone (x 2) to give a light brown solid. This was purified by chromatography on silica gel (100g) eluting with acetone-water (9:1→6:1→7:3). Fractions containing desired product were combined and the solution concentrated by evaporation. The solution was freeze-dried to a light brown solid and this was further dried _in vacuo_ to give _title compound_ (0.22g); $\lambda_{max}$ (ethanol) 234 nm ($\epsilon$ 13,130); $\nu_{max}$ (Nujol) 1770 (β-lactam C=O), 1655 (amide C=O, and τ (DMSO-$d_6$) 5.6-6.0 ($OCH_2$), 8.85-9.35 (cyclopropyl).

_Example 11_

_(6R,7R)-7-[(Z)-2-(2-aminothiazol-4-yl)-2-(α-methoxycarbonylcyclopropylmethoxyimino)acetamido]-3-pyridiniummethylceph-3-em-4-carboxylate dihydrochloride_

The _title compound_ was prepared according to the method of Example 1 using the product of Preparation 19 and exhibited $\lambda_{max}$ (EtOH) 234.5

nm (ε 20,400), $\nu_{max}$ (Nujol) 1778 (β-lactam C=O), 1745 (ester C=O), 1675 $cm^{-1}$ (amide C=O) and τ (DMSO-$d_6$) 3.24, 3.27 (thiazole, mixture of diastereoisomers), 5.98 (-OCH), 9.3-9.6 (cyclopropyl).

0148004

CLAIMS

1. Cephalosporin compounds of general formula (I)

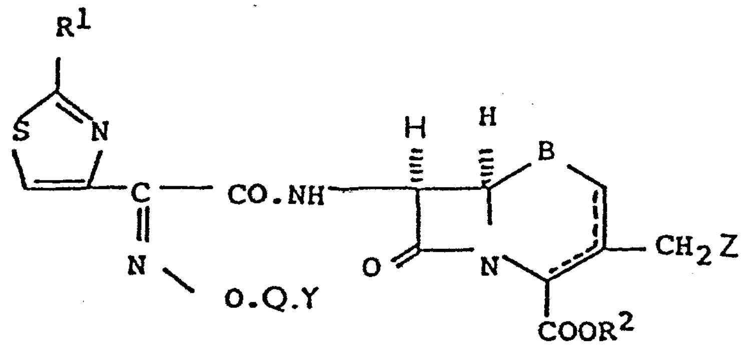

(I)

[wherein

Q represents a cyclopropylmethyl group;

Y represents a carboxyl or blocked carboxyl group, a carbamoyl, N-($C_{1-4}$ alkyl)carbamoyl or N,N-di-($C_{1-4}$ alkyl)carbamoyl group or a cyano group;

Z represents a hydrogen atom; a carbamoyloxy group optionally substituted on the nitrogen atom by a $C_{1-4}$ alkyl or $C_{1-4}$ haloalkyl group; an N-protected carbamoyloxy group; a group of formula $-SZ^a$ (wherein $Z^a$ is a carbon-linked 5- or 6-membered unsaturated heterocyclic ring containing at least one nitrogen atom, which ring may also contain one or more sulphur atoms and/or may be substituted by one or more substituents independently selected from $C_{1-4}$ alkyl, oxo, hydroxy or carbamoyloxymethyl groups); a pyridinium group which may optionally be substituted by one or more substituents independently selected from halogen atoms and carbamoyl, hydroxymethyl and $C_{1-4}$ alkoxy groups; or a bicyclic pyridinium group of formula

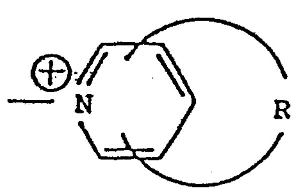

(wherein R represents an optionally substituted $C_{2-5}$ hydrocarbon chain which is saturated or contains one or two double bonds and which may optionally

be interrupted by a heteroatom;

$R^1$ represents an amino or protected amino group;

$R^2$ represents a hydrogen atom or a carboxyl blocking group;

B is -S- or $>S\rightarrow O$ (α- or β-); and the dotted line bridging the 2-, 3- and 4-positions indicates that the compound is a ceph-2-em or ceph-3-em compound] and salts thereof.

2. Cephalosporin antibiotics of general formula (Ia)

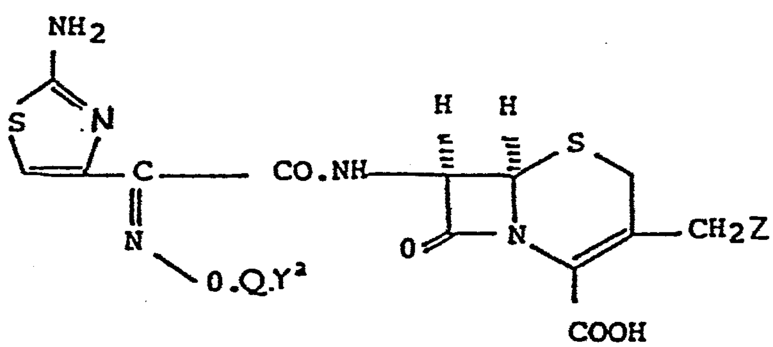

(Ia)

[wherein Q and Z are as defined in claim 1 and $Y^a$ represents a carboxyl, $C_{2-5}$ alkoxycarbonyl, carbamoyl, N-($C_{1-4}$ alkyl)carbamoyl, N,N-di($C_{1-4}$ alkyl)carbamoyl or cyano group] and non-toxic salts and non-toxic metabolically labile esters thereof.

3. Compounds as claimed in claim 2 wherein Z represents a pyridinium group, Q is as defined in claim 1 and $Y^a$ represents a carboxyl, $C_{2-5}$ alkoxycarbonyl, carbamoyl, N-($C_{1-4}$ alkyl)carbamoyl, N,N-di($C_{1-4}$ alkyl)carbamoyl or cyano group.

4. A process for the preparation of compounds as claimed in claim 1, which comprises

(A) acylating a compound of formula (II)

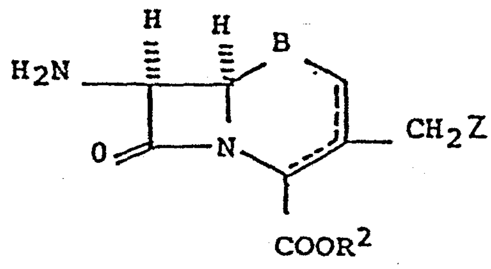

(II)

(wherein Z, B, the dotted line and $R^2$ are as defined in claim 1 or a salt or a 7-N-silyl derivative thereof, with an acid of formula (III)

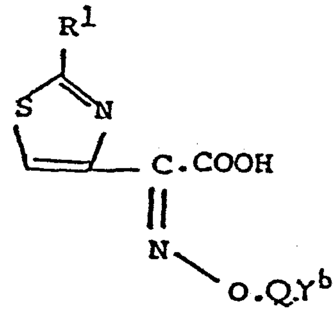

(III)

(wherein $R^1$ and Q are as defined in claim 1, and $Y^b$ is as defined in claim 1 for Y other than a carboxyl group) or a salt thereof, or with an acylating agent corresponding thereto;

(B) for the preparation of compounds wherein Z represents an optionally substituted carbamoyloxy group, reacting a compound of formula (IV)

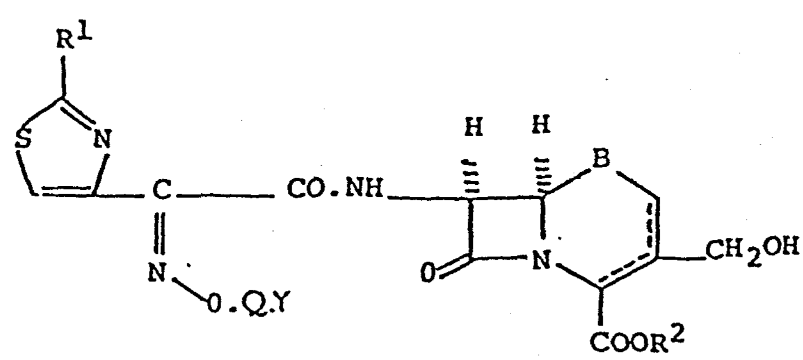

(IV)

(where Q, Y, $R^1$, $R^2$, B and the dotted line are as defined in claim 1) or a salt thereof, with an acylating agent serving to form the group $-CH_2Z^b$ at the 3-position (wherein $Z^b$ is a carbamoyloxy group optionally substituted on the nitrogen atom by a $C_{1-4}$ alkyl or $C_{1-4}$ haloalkyl group or an N-protected carbamoyloxy group);

(C) for the preparation of compounds wherein Z represents an optionally substituted pyridinium or bicyclic pyridinium group or a group of formula $-SZ^a$ (where $Z^a$ is as defined in claim 1), reacting a compound of formula (V)

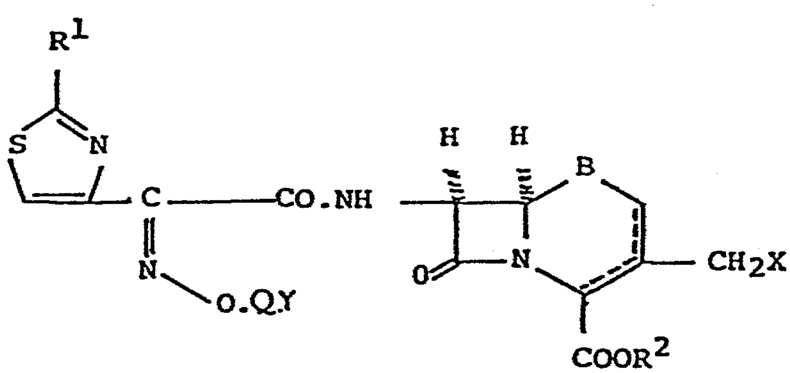

(V)

(wherein Q, Y, $R^1$, $R^2$, B and the dotted line are as defined in claim 1, and X represents a replaceable residue of a nucleophile) or a salt thereof, with a pyridine nucleophile or a sulphur nucleophile serving to form the desired group of formula $-CH_2Z$ at the 3-position; or

(D) for the preparation of compounds wherein Z represents a group of formula $-SZ^a$ wherein $Z^a$ contains a $C_{1-4}$ alkyl-substituted or carbamoylmethyl-substituted nitrogen atom in the heterocyclic ring, reacting a compound of formula (Ib)

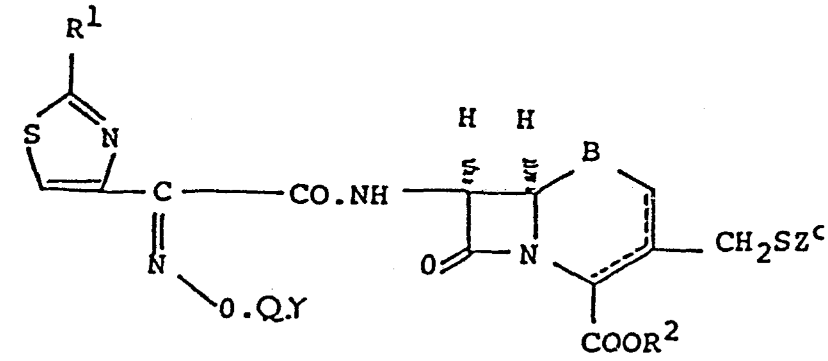

(Ib)

(wherein Q, Y, $R^1$, B and the dotted line are as defined in claim 1; $R^2$ represents a carboxyl blocking group; and $Z^c$ represents a carbon-linked 5- or 6-membered heterocyclic ring containing a nitrogen atom) with a $C_{1-4}$ alkylating agent or a carbamoylmethylating agent serving to introduce a $C_{1-4}$ alkyl group or a carbamoylmethyl group as a substituent on the nitrogen atom in the heterocyclic ring of $Z^c$.

5. A process as claimed in claim 4 for the preparation of compounds according to claim 2, wherein, if necessary and/or desired in each instance, any of the following reactions, in any appropriate sequence, are carried out subsequent to reaction (A), (B), (C) or (D):

i) conversion of a $\Delta^2$-isomer into a $\Delta^3$-isomer,
ii) reduction of a compound wherein B is $>S\rightarrow O$ to form a compound wherein B is -S-,
iii) conversion of a carboxyl group into a non-toxic metabolically labile ester function,
iv) formation of a non-toxic salt,
v) conversion of one group Y into another group Y, and
vi) removal of any carboxyl blocking and/or N-protecting groups.

6. A pharmaceutical composition comprising as active ingredient a compound according to claim

2 in association with one or more pharmaceutical carriers or excipients.

7. Compounds of general formulae (III), (IV) and (V) as defined in claim 4 and salts, esters and reactive derivatives thereof.